# EUROPÄISCHE PATENTANMELDUNG

(11) **EP 0 714 646 A1**
(43) Veröffentlichungstag der Anmeldung: **05.06.1996**
(21) Anmeldenummer: 95118889.5
(22) Anmeldetag: 30.11.1995
(51) Int. Cl.: A61F 9/00

(54) **Vorrichtung zum Abtragen von Gewebe an einer Augenhornhaut**

(30) Priorität: 30.11.1994 DE 4442669
(71) Anmelder: Herbert Schwind GmbH & Co. KG, D-63801 Kleinostheim (DE)
(72) Erfinder: Seiler Theodor Prof. dr.dr. Augenklinik-Univ-, D-01307 Dresden (DE); Borrmann Andreas, D-55218 Ingelheim (DE)
(74) Vertreter: Nöth, Heinz, Dipl.-Phys.

(57) **Zusammenfassung**

Eine Vorrichtung zum Abtragen von Gewebe an einer Augenhornhaut mit einem Laserstrahlgenerator zur Erzeugung einer Laserstrahlung, einer optischen Einrichtung, durch welche bei der Gewebeabtragung die Laserstrahlung mit einem etwa kreisrunden Bestrahlungsquerschnitt und einem bestimmten Abtragungsprofil der auf das abzutragende Hornhautgewebe übertragene Strahlungsenergie auf die zu behandelnde Hornhaut gerichtet wird, und einer Steuereinrichtung zur Steuerung der optischen Einrichtung dahingehend, daß ein Abtragungsprofil gebildet wird, das im radialen Schnitt, ausgehend von der Strahlungsmittelachse 2 des Bestrahlungsquerschnitts in einem inneren Bereich 3 einen etwa sphärischen bzw. konkaven Verlauf, der in einen Wendepunkt 4 übergeht, und in einem an den Wendepunkt 4 sich anschließenden äußeren radialen Bereich 5 mit einem konvexen bzw. monoton fallenden Verlauf aufweist.

## Beschreibung

Die Erfindung betrifft eine Vorrichtung zum Abtragen von Gewebe an einer Augenhornhaut mit einem Laserstrahlgenerator zur Erzeugung einer Laserstrahlung, einer optischen Einrichtung, durch welche bei der Gewebeabtragung die Laserstrahlung auf die zu behandelnde Hornhaut mit einem Bestrahlungsquerschnitt und einem bestimmten Abtragungsprofil der auf das abzutragene Hornhautgewebe übertragenen Strahlungsenergie gerichtet wird, und einer Steuereinrichtung zur Steuerung der optischen Einrichtung.

Eine derartige Vorrichtung ist aus der DE 41 03 615 C2 bekannt. Die Vorrichtung dient zur Beseitigung von Fehlsichtigkeit, die sich insbesondere infolge von Refraktionsanomalie des Auges ergibt. Dabei wird auf die Hornhaut ein Laserstrahl gerichtet, der über seinen Querschnitt hin ein Abtragungsprofil, d.h. ein auf das abzutragende Hornhautgewebe zu übertragendes Strahlungsenergieprofil im Bestrahlungsquerschnitt hat. Problematisch gestaltet sich bei den bisher bekannt gewordenen chirurgischen Behandlungsvorrichtungen, insbesondere bei der Myopiekorrektur mit hohen Scheitelbrechwerten, insbesondere über sechs Dioptrien, daß die Hornhaut mit einer relativ hohen Abtragungstiefe behandelt werden muß, um eine ausreichende Korrektur der Refraktionsanomalie zu erhalten. Hieraus ergibt sich das Risiko der Narbenbildung im postoperativen Heilungsverlauf, insbesondere am Rand des Abtragungsfeldes. Vor allem bei erweiterter Pupillenöffnung bei schlechter Beleuchtung oder Dämmerlicht bzw. in der Nacht beeinträchtigt die Narbe das Sehvermögen.

Aufgabe der Erfindung ist es, eine Vorrichtung der eingangs genannten Art zu Schaffen, bei der eine erhebliche Verringerung der Abtragungstiefe bei der Korrektur von Refraktionsanomalie, insbesondere von Myopie, erreicht wird.

Diese Aufgabe wird erfindungsgemäß dadurch gelöst, daß das von der optischen Einrichtung gebildete Abtragungsprofil im Radialschnitt, ausgehend von der Strahlungsmittelachse des Bestrahlungsquerschnitts, in einem inneren radialen Bereich einen etwa sphärischen oder konkaven Verlauf, der in einem Wendepunkt übergeht, und einen an den Wendepunkt sich anschließenden äußeren radialen Bereich mit einem konvexen bzw. monoton fallenden Verlauf aufweist.

Durch das so ausgebildete Abtragungsprofil des auf das abzutragende Hornhautgewebe vom Laserstrahl übertragenen Strahlungsenergie innerhalb des Bestrahlungsquerschnitts wird eine erhebliche Verringerung der Abtragungstiefe erreicht.

Der Wendepunkt des Abtragungsprofils kann, ausgehend von der Strahlungsmittelachse, bei etwa 40% bis 60%, insbesondere etwa 50%, der jeweiligen radialen Ausdehnung des Bestrahlungsquerschnitts liegen.

In bevorzugter Weise wird die optische Einrichtung, in welcher das Abtragungsprofil eingestellt wird, mit einem Laserstrahl beschickt, der über einen Strahlquerschnitt hin eine gleichbleibende Intensitätsverteilung hat. Zur Erzeugung der gleichbleibenden Intensitätsverteilung kann beispielsweise ein Strahlintegrator verwendet werden, wie er aus der DE 41 03 615 C2 bekannt ist.

Man erreicht gegenüber bekannten etwa sphärisch ausgebildeten Abtragungsprofilen eine Verringerung der Abtragungstiefe von ungefähr 30%. Da hierdurch die Gefahr der Narbenbildung, insbesondere im Randbereich, erheblich verringert ist, können Abtragungsdurchmesser zwischen 6 und 8 mm bei der Hornhautübertragung durchgeführt werden.

Der Strahlungsquerschnitt kann kreisrund, annähernd kreisrund und für Astigmatismuskorrektur oval oder etwa elliptisch sein.

Zur Erzielung des gewünschten Abtragungsprofils kann eine Blendeneinrichtung verwendet werden, die auf der zu behandelnden Gewebefläche gleichzeitig mehrere Strahlflecke bildet. Diese Strahlflecke können kreisrund sein und unterschiedliche Durchmesser aufweisen. Ferner ist es möglich, Strahlflecke mit regelmäßiger, z.B. polygonaler Gestalt (z.B. Sechseck, Achteck) oder unregelmäßiger Geometrie, die beispielsweise aus Fraktals zusammengesetzt ist, zum Einsatz zu bringen. Die so gebildeten Strahlflecke bzw. der so ausgebildete Strahlfleck kann durch Drehen der Blendenöffnung bzw. des auf dem Gewebe gebildeten Strahlflecks die gewünschte Strukturierung des Hornhautgewebes mit dem erfindungsgemäßen Abtragungsprofil erzeugen. Eine derartige Vorrichtung ist aus der EP 0 651 082 A1 bekannt. Die Wellenlänge der einzusetzenden Laserstrahlung liegt im kurzwelligen Ultraviolettbereich (EP-A-0 111 060 oder Trokel et al: _{"}Excimer Laser Surgery of the Cornea" in American Journal of Ophthalmology Dec. 1983, Band 96: 710-715).

Auch ist es möglich, einen Strahlungsfleck zu bilden, der über die zu behandelnde Gewebefläche geführt wird. Dabei wird im wesentlichen unter Einsatz der bei der bekannten Vorrichtung vorhandenen Mittel über die zu behandelnde Gewebefläche, insbesondere Stroma, ein Laserstrahl geführt, der auf der Gewebefläche einen Strahlfleck bildet mit einem Bruchteil der Flächenausdehnung der zu behandelnden Gewebefläche. Die Flächenausdehnung des Strahlflecks beträgt etwa 1/30 bis 1/10 der zu behandelnden Gewebefläche. Ferner hat der auf dem zu behandelnden Gewebe gebildete Strahlungsfleck insbesondere an seinem Rand eine regelmäßige polygonale oder unregelmäßige geometrische Form. Diese unregelmäßig geometrische Form kann gebildet sein aus mehreren gleichzeitig gebildeten kreisrunden Flecken unterschiedlicher Durchmesser und unregelmäßiger Anordnung oder durch einen insbesondere sternförmigen Fleck mit ungeradlinigen Öffnungsbegrenzungskanten oder einen Fleck mit unregelmäßigen Verästelungen, die durch fraktale Geometrieelemente gemäß der mathematischen Theorie nach B. Mandelbrot _{"}Les objets fractals, Editions Flammarion, Paris, 1974 - 1984" und _{"}The fractal geometry of nature, Editions W.H. Freeman and Company, San Francisco 1977 - 1982" gebildet sind. Um diese geometrische Form des auf der Gewebefläche gebildeten Strahlenflecks zu erhalten, wird der Öffnungsbereich der Blendeneinrichtung entsprechend ausgebildet. Hierzu können, wie in der EP 0 651 082 A1 gezeigt ist, mehrere kreisrunde Blendenöffnungen vorgesehen sein, oder es kann eine Blendenöffnung vorgesehen sein, welche an ihrem Rand Verästelungen und Verzweigungen aufweist bzw. in unregelmäßiger Sternform ausgebildet ist.

Dieser so gebildete und gedrehte Strahlfleck wird mit Hilfe einer Ablenkeinrichtung über die zu behandelnde Gewebeoberfläche geführt. Aus der EP 0 151 869 A1 ist es zwar bekannt, mit dem von der Laserstrahlquelle gelieferten Laserstrahl auf der zu behandelnden Gewebeoberfläche einen Strahlfleck zu bilden und mit diesem den zu behandelnden Gewebebereich abzutasten. Jedoch wird keine Blende verwendet, mit welcher der Strahlfleck in eine unregelmäßige, insbesondere durch fraktale Geometrieelemente gebildete Geometrieform gebracht wird.

Während der Bestrahlung, welche insbesondere impulsförmig ist, wird der Strahlfleck um seine Mitte (Achse) gedreht. Es kann dadurch geschehen, daß der die Blende verlassende Laserstrahl mit Hilfe eines oder mehrerer Prismen oder Spiegel gedreht wird. Ohne zusätzlichen Aufwand läßt sich die Drehung dadurch erreichen, daß die Blendenöffnung gedreht wird. In jeder Abtast- bzw. Ablenkstellung können ein oder mehrere, insbesondere zwei bis zehn Strahlungsimpulse erzeugt werden. Die Ablenkung bzw. Abtastung des Strahlungsflecks erfolgt gegenüber einer beispielsweise von einem Fixationslaser (EP 0 651 082 A1) gebildeten Fixierlinie, entlang welcher das zu behandelnde Auge mit seiner Gesichtslinie ausgerichtet ist. Die Auslenkung kann dabei in einer zur Fixierlinie senkrecht liegenden Ebene nach X- und Y-Koordinaten erfolgen oder durch Verschwenken des auf das Hornhautgewebe gerichteten Laserstrahls bezüglich der Fixierlinie.

Bei der erfindungsgemäßen Vorrichtung kann eine Laserstrahlquelle mit verringerter Laserausgangsleistung verwendet werden. Durch die insbesondere am Rand des Blendenöffnungsbereichs unregelmäßige, insbesondere durch fraktale Geometrieelemente gebildete Strahlgeometrie können mit Hilfe einer insbesondere sternförmig mit regelmäßiger oder unregelmäßiger, insbesondere fraktaler Randgestaltung ausgebildeten Blendenöffnung praktisch alle Typen von Sehkorrekturen erreicht werden. Insbesondere können Astigmatismuskorrekturen und hier vor allem irreguläre Astigmatismen auch korrigiert werden. Im Behandlungsbereich des Laserstrahls wird ein abgeflachter Verlauf der behandelten Oberfläche gegenüber der nicht behandelten Öberfläche erreicht.

Die Vorrichtung kann nicht nur für eine Abtragung der Hornhaut an der Hornhautaußenseite durchgeführt werden, sondern auch zur Abtragung von Gewebe im Stroma.

Zur weiteren Erläuterung der Erfindung dienen die Figuren. Es zeigt:
- Fig. 1:: schematisch ein Ausführungsbeispiel der Erfindung;
- Fig. 2:: ein Abtragungsprofil der auf das zu behandelnde Auge gerichteten Laserstrahlung;
- Fig. 3:: in mehr detailierter schematischer Darstellung eine Gesamtansicht einer Vorrichtung, welche ein Ausführungsbeispiel der Erfindung ist;
- Fig. 4:: eine beim Ausführungsbeispiel einsetzbare Blende;
- Fig. 5:: eine weitere Ausführungsform einer Blende;
- Fig. 6:: eine erste Ausführungsform für eine Strahlablenkeinrichtung;
- Fig. 7:: eine zweite Ausführungsform für eine Strahlablenkeinrichtung; und
- Fig. 8:: ein Abtragungsprofil, welches erreicht wird.

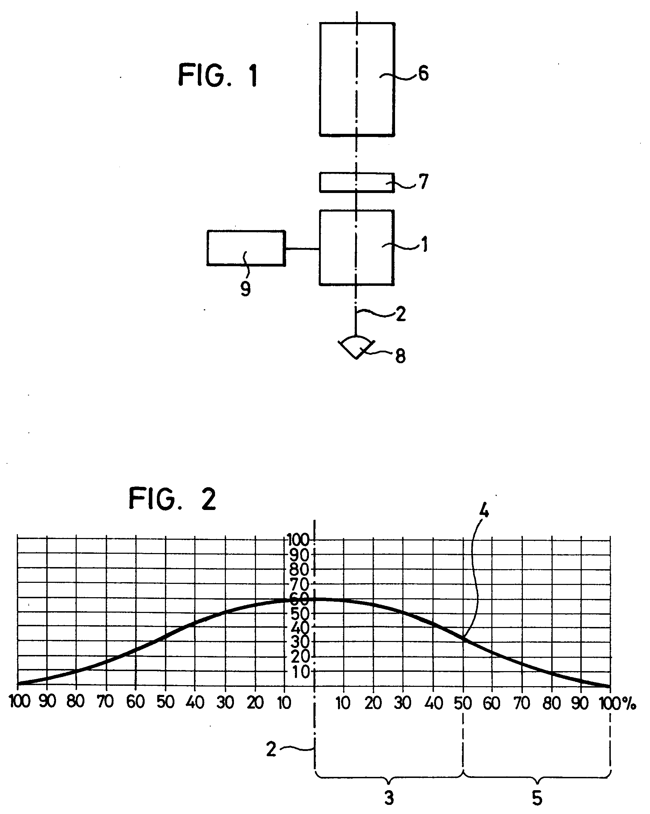

Das in der Figur 1 dargestellte Ausführungsbeispiel besitzt eine Laserstrahlquelle, beispielsweise in Form eines Eximerlasers, der Laserstrahlung bei einer Wellenlänge, insbesondere von 193 Nanometer, aussendet, so daß abzutragendes Hornhautgewebe zersetzt und abgetragen wird. Auch Wellenlängen von 199 nm und 208 nm sind geeignet. Diese Laserstrahlquelle bildet einen Lasergenerator 6. Die Strahlung des Lasergenerators 6 wird mit Hilfe eines Strahlungsintegrators 7 homogenisiert, so daß über den Strahlungsquerschnitt hin, der in eine optische Einrichtung 1 eingeleitet wird, eine im wesentlichen konstante Intensitätsverteilung hergestellt wird. Der Strahlungsintegrator kann in der Weise ausgebildet sein, wie es aus der DE 41 03 615 C2 bekannt ist.

In dem Strahlgang mit homogener Intensitätsverteilung im Strahlungsquerschnitt befindet sich die optische Einrichtung 1, welche ein Abtragungsprofil erzeugt, wie es in der Fig. 2 im Radialschnitt durch eine Strahlungsmittelachse 2 dargestellt ist. Hierzu kann die optische Einrichtung 1 ein Belichtungssystem aufweisen, mit dem in den jeweiligen radialen Bereichen des Bestrahlungsquerschnitts, der den Behandlungsquerschnitt an der Hornhaut eines zu behandelnden Auges 8 bildet, unterschiedliche Belichtungszeiten mit der homogenen Laserstrahlung in der Weise einstellt werden. Das in der Fig. 2 dargestellte Profil der von der Strahlung auf das abzutragende Hornhautgewebe übertragenen Strahlungsenergie hat ein entsprechendes Belichtungszeitprofil. Dieses Abtragungsprofil entspricht jeweiligen radialen Schnitten durch eine Strahlungsmittelachse 2 des behandelnden Laserstrahls, der aus der optischen Einrichtung 1 austritt und auf die Hornhaut des zu behandelnden Auges 8 auftrifft.

Die sich über den Bestrahlungsquerschnitt für unterschiedliche Radiusbereiche ändernde zu übertragende Strahlungsenergie (Abtragungsprofil) kann zur Erzielung entsprechender Belichtungszeiten in den jeweiligen radialen Bereichen durch sich ändernde Blendenöffnungen im homogenen Strahlungsquerschnitt, gegebenenfalls in Verbindung mit einem Linsensystem, z.B. Zoom-Linsensystem, erreicht werden. Eine derartige Vorrichtung ist beispielsweise aus der DE 41 03 615 C2 bekannt.

Es ist jedoch auch möglich, anstelle der Steuerung der Belichtungszeit den Laserstrahl mit homogener Intensitätsverteilung über seinen Bestrahlungsquerschnitt hin durch ein Filter zu schicken, mit dem ein Intensitätsprofil erzeugt wird, das dem Abtragungsprofil der Fig. 2 entspricht.

Zur Einstellung des entsprechenden Intensitätsprofils bzw. Belichtungszeitprofils zur Erzielung des in Fig. 2 dargestellten Abtragungsprofils kann die optische Einrichtung 1 mit einer Steuereinrichtung 9 verbunden sein, die eine elektronische Processoreinrichtung aufweist. In die Steuereinrichtung 9 werden die erforderlichen Angaben für die Korrektur der Refraktionsanomalie eingegeben, beispielsweise der entsprechende Scheitelbrechwert des Radius des Behandlungsquerschnitts (Bestrahlungsquerschnitt). Die Steuereinrichtung besitzt ferner einen Speicher, in welchem das Abtragungsprofil der Fig. 2 gespeichert ist. Dieses Abtragungsprofil wird dann zusammen mit den jeweils für die Refraktionskorrektur eingegebenen Daten zur Steuerung der optischen Einrichtung 1 verwendet.

Die Fig. 2 zeigt einen Radialschnitt durch ein Abtragungsprofil um die Strahlungsmittelachse 2. Auf der Koordinate ist die auf das abzutragende Hornhautgewebe zu übertragende Strahlungsenergie aufgetragen. Diese Strahlungsenergie bewirkt in entsprechenden radialen Bereichen des Bestrahlungsquerschnitts die gewünschten Abtragungstiefen. Auf der Abszisse ist in Prozentangaben der jeweilige Radius des Bestrahlungsquerschnitts aufgetragen. Insofern kann das Radialschnittprofil auf jeweilige erforderliche Radien des Bestrahlungsquerschnitts angewendet werden. Der Bestrahlungsquerschnitt kann etwa kreisrund ausgebildet sein, jedoch können auch hiervon abweichende Querschnittsformen je nach der anzuwendenden Refraktionskorrektur zur Anwendung kommen, z.B. ovale oder etwa elliptische Formen, insbesondere bei Astigmatismuskorrektur.

Das in der Fig. 2 dargestellte Radialschnittprofil besitzt, ausgehend von der Strahlungsmittelachse 2, einen inneren radialen Bereich 3 mit etwa sphärischem bzw. konkavem Verlauf. Dieser Profilkurvenverlauf geht über in einen Wendepunkt 4. Der Wendepunkt 4 liegt beim dargestellten Ausführungsbeispiel etwa bei 50% des Radius des Bestrahlungsquerschnitts. An den Wendepunkt 4 schließt sich ein äußerer radialer Berich 5 an mit einem konvexen bzw. monoton fallenden Verlauf, der bei 100 % des Radius des Bestrahlungsquerschnitts endet.

Gegenüber bekannten Abtragungsprofilen erreicht man eine etwa 30%ige Abflachung bzw. Verringerung der Abtragungstiefen bei gleicher effektiver Korrektur der Refraktionsanomalie, insbesondere bei der Myopiekorrektur. Z.B. war bei einer Kurzsichtigkeit von 9 dpt. bisher eine Abtragungstiefe von 108 µm in der Mitte erforderlich. Diese Abtragungstiefe verringert sich bei Anwendung eines erfindungsgemäßen Abtragungs- bzw. auf die Hornhaut übertragenen Strahlungsenergieprofils auf 71 µm.

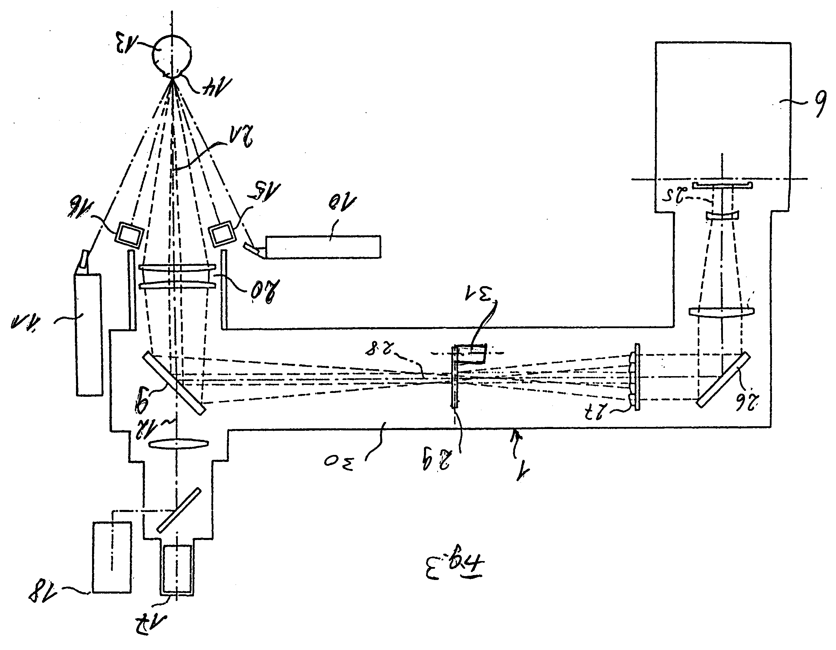

Das in der Figur 2 dargestellte Strahlungsenergieprofil läßt sich z.B. mit der in der Fig. 3 dargestellten Vorrichtung realisieren. Diese besitzt im wesentlichen die gleichen Bauteile wie die in der EP 0 651 082 A1 dargestellte Vorrichtung. Die Laserstrahlquelle 6 sendet einen Laserstrahl 25 aus, der über eine Umlenkeinrichtung, welche beim dargestellten Ausführungsbeispiel als Umlenkspiegel 26 ausgebildet ist, durch einen Strahlhomogenisator 27 hindurchgeleitet wird. Der Strahlhomogenisator 27 kann in der Weise ausgebildet sein, wie es aus der DE 41 03 615 C2 bekannt ist. Durch den Strahlhomogenisator wird über den Strahlquerschnitt hin eine einheitliche Strahlungsintensität erzeugt.

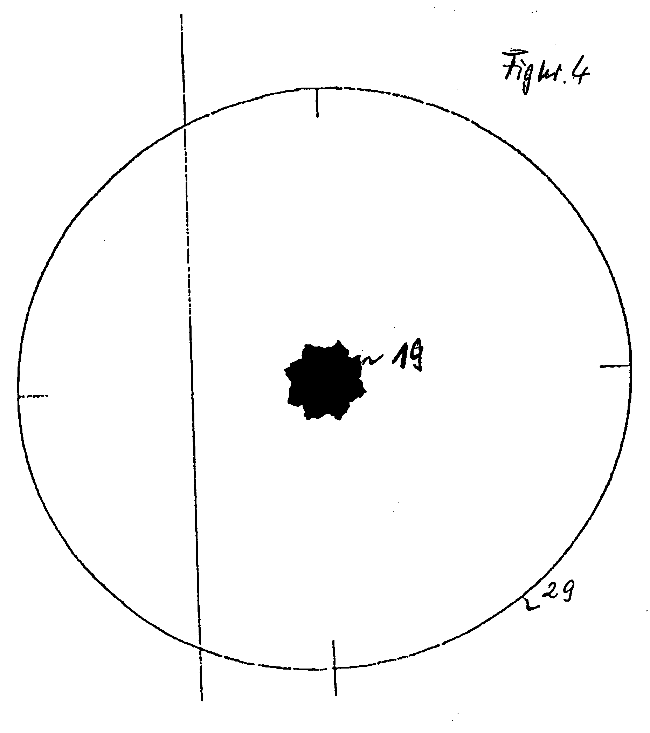

Im Strahlengang des homogenisierten Laserstrahls mit der Strahlachse 28 befindet sich eine Blendeneinrichtung 29. Die Blendeneinrichtung 29 kann so ausgebildet sein, wie es in der EP 0 651 082 A1 beschrieben ist. Ausführungsbeispiele dieser Blendeneinrichtung 29 sind ferner in den Figuren 4 und 5 dargestellt.

Durch die Blendeneinrichtung 29 wird der homogene Laserstrahl geformt. Der Laserstrahl weist einen Querschnitt auf, der dem Öffnungsbereich der Blendeneinrichtung 29, welcher durchlässig ist für die Laserstrahlung, entspricht. Dieser geformte Laserstrahl wird über eine weitere Umlenkeinrichtung, welche als halbdurchlässiger Umlenkspiegel 9 ausgebildet sein kann, durch ein Objektivlinsensystem 20 auf eine zu behandelnde Hornhaut 14 eines Patientenauges 13 gerichtet. Die Laserstrahlquelle und die zur Modifizierung und Führung des Laserstrahls vorgesehenen optischen Komponenten sind in einem Gehäuse 6 angeordnet.

Ferner ist ein Fixierziel in Form eines Fixierlasers 17 vorgesehen, auf welches der Patient mit seinem zu behandelnden Auge 13 blickt. Der Fixierlaser 17 bildet eine Fixierlinie 12. Entlang dieser Fixierlinie 12 ist die Gesichtslinie des Patientenauges 13 während der Behandlung ausgerichtet. Die Fixierlinie 12 wird durch einen Laserstrahl des Fixierlasers 17 gebildet. Dieser Fixerungslaserstrahl wird auf der Augenhornhautoberfläche in Form eines Strahlungsfleckes abgebildet, und mit Hilfe von Pilotlasern 10 und 11 erfolgt die Ausrichtung des für die Behandlung der Augenhornhaut 14 verwendeten Behandlungslaserstrahls 21, welcher durch die Blendeneinrichtung 29 und das Objektivlinsensystem 20 in eine geeignete Form gebracht ist, ausgerichtet. Durch Okulare 15 und 16 kann die genaue Ausrichtung des Behandlungslaserstrahls 21 mit der Fixierlinie 12 beobachtet werden.

Ferner ist eine Überwachungseinrichtung 18, beispielsweise in Form eines Monitors, vorgesehen, welcher den Behandlungsablauf überwacht. Eine Abschaltung wird dann vorgenommen, wenn der Behandlungslaserstrahl 21 nicht mehr mit der Fixierlinie 12 ausgerichtet ist, wie es beispielsweise in der DE 42 32 021 A1 beschrieben ist.

Die Blende 29 kann, wie in Fig. 4 dargestellt ist, einen Blendenöffnungsbereich in Form einer etwa sternförmigen Blendenöffnung 19 aufweisen. Die sternförmige Blendenöffnung 19 besitzt ungeradlinig verlaufende Begrenzungskanten, deren Geometrie bevorzugt durch fraktale Geometrieelemente gebildet ist. Die Blendenöffnung 19 ist in der Mitte der Blende 29 angeordnet. Der Durchmessser der Blendenöffnung beträgt ca. 2,2 mm. Der auf der zu behandelnden Gewebefläche gebildete Strahlfleck hat einen Durchmesser von ca. 1,75 mm. Dieser Strahlfleck hat eine Querschnittsform, die der Blendenöffnung 19 entspricht.

Bei dem in Figur 5 dargestellten Ausführungsbeispiel einer Blendenöffnung besitzt die Blendenöffnung 19 eine regelmäßige geometrische Gestalt in Form eines Polygons (Sechseck beim dargestellten Ausführungsbeispiel). Es kann auch eine regelmäßige Sternform mit geradlinig verlaufenden Kanten als Blendenöffnung verwendet werden. Auch andere Vielecke, z.B. Viereck, Achteck, sind als geometrische Formen der Blendenöffnung möglich.

Während der Behandlung wird die Querschnittsform des auf dem Gewebe gebildeten Strahlungsflecks gedreht. Dies erfolgt beim Ausführungsbeispiel der Fig. 3 dadurch, daß die Blende 29 durch eine Dreheinrichtung 31 gedreht wird. Die Dreheinrichtung 31 und die Blende 29 können hierzu auch so ausgebildet sein, wie es in der EP 0 651 082 A1 beschrieben ist.

Da im Falle der Blendenbeispiele der Figuren 4 und 5 ein relativ geringer Durchmesser der Blendenöffnung 19 bei der Erfindung erforderlich ist, genügt es, wenn die Laserstrahlvorrichtung 6 einen Laserstrahl 25 mit relativ geringem Durchmesser aussendet. Der vom Homogenisator 27 erzeugte homogene Strahl kann am Ort der Blende 29 ebenfalls einen relativ geringen Durchmesser mit homogener Strahlverteilung bzw. Intensität aufweisen.

Durch Drehen des auf dem zu behandelnden Augenhornhautgewebe gebildeten Strahlfleckes kann eine Abtragungsstruktur erreicht werden, wie sie in der Fig. 8 gezeigt ist. Die in der Fig. 8 gezeigte Abtragungsstruktur ist erzeugt durch eine impulsförmige Laserstrahlung mit einer Querschnittsform der Blendenöffnung 19 und einen Strahlfleckdurchmesser von 1,75 mm auf der Gewebeoberfläche. Mit Hilfe von drei Strahlungsimpulsen wurde eine Abtragungstiefe von etwa 0,75 Mikrometer erreicht. Die Abtragungstiefe ist über einen Strahlungsdurchmessser von ca. 1,2 mm konstant und hat am Rand einen abgeschrägten Profilverlauf mit einem Wendepunkt, wie es beispielsweise in der Figur 2 gezeigt ist. Durch dieses Abtragungsprofil läßt sich am Rand des behandelten Augenhornhautbereiches gegenüber dem nicht behandelten Augenhornhautgewebe ein kontinuierlicher Übergang schaffen. Ferner erreicht man eine verringerte Abtragungstiefe bei der Refraktionskorrektur.

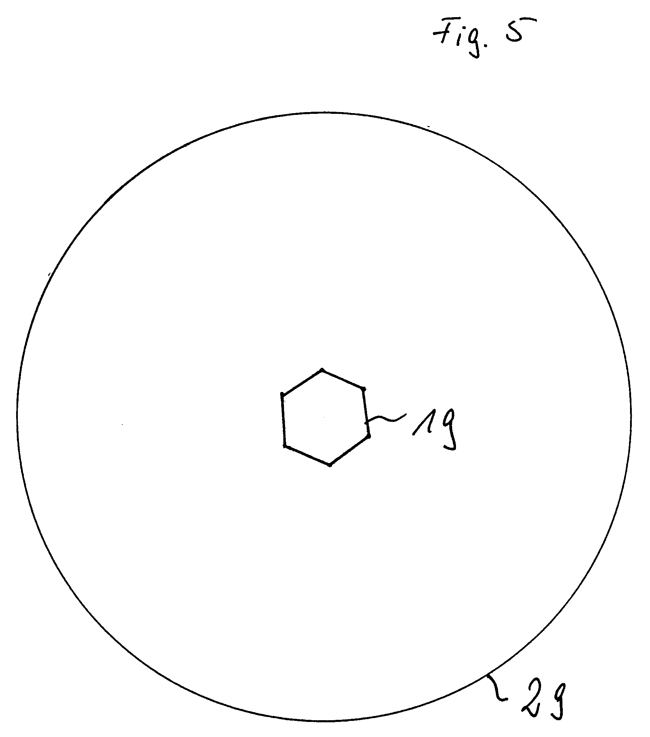

Der gedrehte Laserstrahlfleck tastet während der Behandlung die gesamte Fläche des zu behandelnden Gewebes ab. Dabei werden in Abhängigkeit von der zu erzielenden Abtragungstiefe an den verschiedenen Gewebeflächenteilen eine entsprechende Anzahl an Laserstrahlungsimpulsen zur Einwirkung gebracht. Zur Führung des Laserstrahlungsflecks über die zu behandelnde Gewebefläche ist eine Ablenkeinrichtung für den Behandlungslaserstrahl 21 vorgesehen. Ausführungsbeispiele für eine derartige Strahlablenkungseinrichtung sind in den Figuren 6 und 7 dargestellt.

Bei dem in der Figur 6 dargestellten Ausführungsbeispiel wird der Umlenkspiegel 9 zur Strahlfleckbewegung über die zu behandelnde Gewebeoberfläche um einen Drehpunkt 23 bewegt. Dieser Drehpunkt 23 wird gebildet durch den Schnittpunkt der Fixierlinie 12 mit der reflektierenden Oberfläche des Ablenkspiegels 9, an welchem der Behandlungslaserstrahl 21 reflektiert wird. Mit Hilfe einer entsprechend ausgebildeten Antriebseinrichtung 22 und einer entsprechenden Kugelgelenk- oder kardanischen Aufhängung des Umlenkspiegels 9 läßt sich die gewünschte Auslenkung bzw. Abtastbewegung des Behandlungslaserstrahls 21 auf dem Augenhornhautgewebe erreichen. Diese Bewegung erfolgt um den Drehpunkt 23, wobei der Behandlungslaserstrahl 21 gegenüber der fest bleibenden Fixierlinie 12 in der gewünschten Weise ausgelenkt wird. Die Auslenkung erfolgt dabei in einem räumlichen Winkelbereich.

Bei dem in der Fig. 7 dargestellten Ausführungsbeispiel wird das Objektivlinsensystem 20 mit Hilfe einer x,y-Antriebseinrichtung 24 in einer zur Fixierlinie 12 senkrechen Ebene zur gewünschten Ablenkung des Behandlungslaserstrahls 21 bewegt. Es ist natürlich auch möglich, ein zusätzliches optisches Ablenksystem vorzusehen, welches mit der x,y-Antriebseinrichtung 24 in der zur Fixierlinie 12 senkrechten Ebene bewegt wird.

Die beiden in den Figuren 6 und 7 dargestellten Ablenkeinrichtungen können auch in Kombination miteinander verwendet werden, insbesondere zur Korrektur von Astigmatismus. Es ist jedoch auch möglich, die Astigmatismuskorrektur jeweils mit einer der beiden Ablenkeinrichtungen der Figuren 3 und 4 zu erreichen.

Anstelle der in den Figuren 4 und 5 gezeigten Blendenöffnungen können zur Querschnittsbildung des Behandlungslaserstrahls 21 auch andere Blendenöffnungsformen verwendet werden. Diese Blendenöffnungsformen können dabei in der Weise ausgebildet sein, daß sie von mehreren kreisförmigen Blendenöffnungen gebildet werden, wie sie beispielsweise in den Figuren 6 bis 8 der EP 0 651 082 A1 gezeigt sind. Diese kreisrunden unterschiedliche Durchmesser aufweisenden Blendenöffnungen können auch in einem engen Durchmesser mit gegenüber der Blende in der EP 0 651 082 A1 verringerter Durchtrittsöffnung zentrisch um die Blendenmitte bzw. um die Drehachse der Blende angeordnet sein. Bevorzugt wird hierbei eine Blendenöffnungsform, die der Fig. 6 der EP 0 651 082 A1 entspricht.

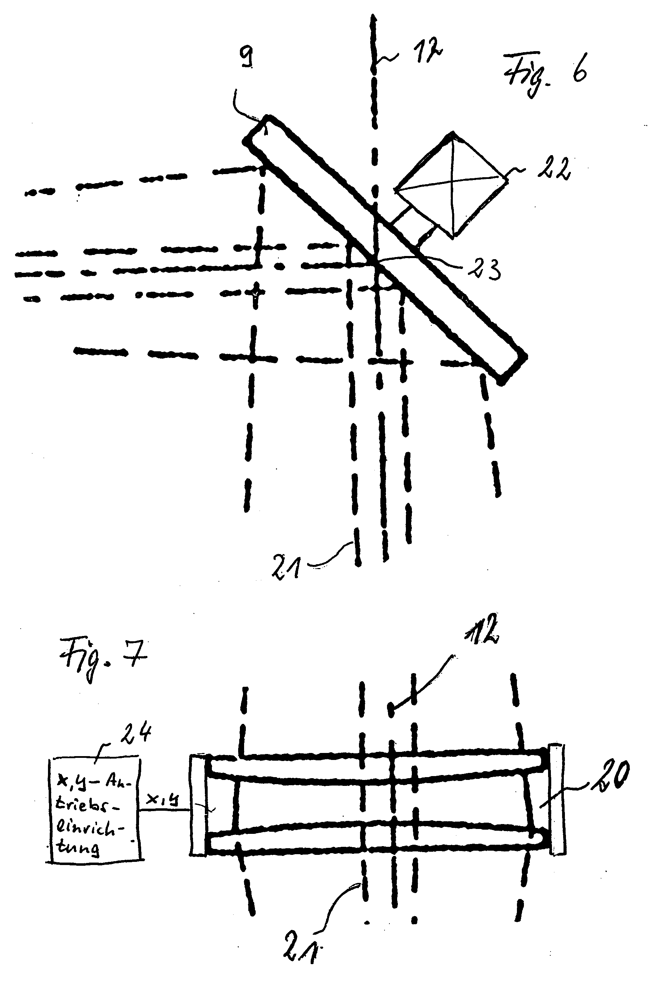

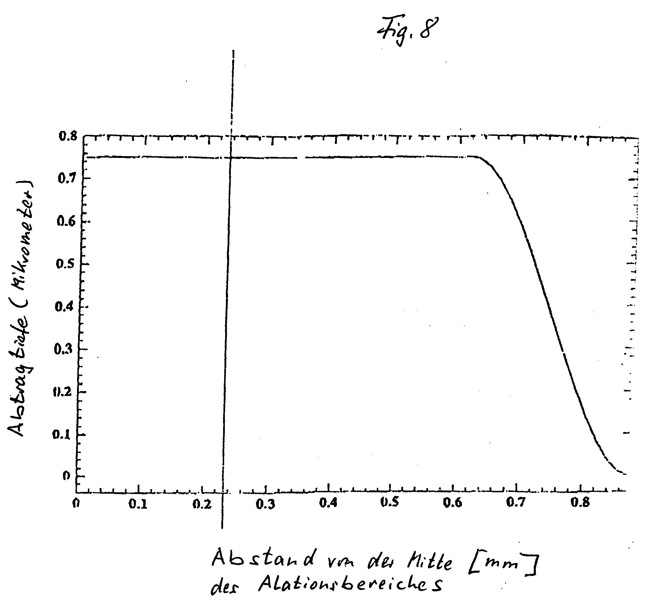

Ferner können Blendenöffnungsformen verwendet werden, wie sie in den Figuren 9 bis 14 der EP 0 651 082 A1 gezeigt sind. Die Blendenöffnungen besitzen jedoch eine verringerte Blendenöffnungsfläche. Gegenüber der Fig. 11 besitzt die Blendenöffnung eine um 5 bis 15 % verringerte Öffnungsfläche.

Die Erfindung ist nicht nur einsetzbar bei der Bestrahlung der Augenhornhaut, sondern sie kann auch eingesetzt werden zur Behandlung des Stroma-Gewebes der Augenhornhaut nach teilweise oder ganzem Abheben einer Hornhautlamelle und Wiederzurückbringen der abgehobenen Augenhornhautlamelle auf das behandelte Stroma-Gewebe.

## Patentansprüche

1. Vorrichtung zum Abtragen von Gewebe an einer Augenhornhaut mit einem Laserstrahlgenerator zur Erzeugung einer Laserstrahlung, einer optischen Einrichtung, durch welche bei der Gewebeabtragung die Laserstrahlung auf die zu behandelnde Hornhaut mit einem Bestrahlungsquerschnitt und einem bestimmten Abtragungsprofil der auf das abzutragende Hornhautgewebe übertragenen Strahlungsenergie gerichtet wird, und einer Steuereinrichtung zur Steuerung der optischen Einrichtung,
dadurch **gekennzeichnet**,
daß das von der optischen Einrichtung (1) gebildete Abtragungsprofil im Radialschnitt, ausgehend von der Strahlungsmittelachse (2) des Bestrahlungsquerschnittes in einem inneren radialen Bereich (3) einen etwa sphärischen bzw. konkaven Verlauf, der in einen Wendepunkt (4) übergeht, und einen an den Wendepunkt (4) sich anschließenden äußeren radialen Bereich (5) mit einem konvexen bzw. monoton fallenden Verlauf aufweist.

2. Vorrichtung nach Anspruch 1, dadurch gekennzeichnet, daß der Wendepunkt (4), ausgehend von der Strahlungsmittelachse (2) bei etwa 40% bis 60% der radialen Ausdehnung des Bestrahlungsquerschnitts liegt.

3. Vorrichtung nach Anspruch 1 oder 2, dadurch gekennzeichnet, daß zwischen dem Laserstrahlgenerator (6) und der optischen Einrichtung (1) ein Strahlintegrator (7) angeordnet ist, der eine gleichbleibende Intensitätsverteilung über den in die optische Einrichtung (1) eingeleiteten Bestrahlungsquerschnitt hin erzeugt.

4. Vorrichtung nach einem der Ansprüche 1 bis 3, dadurch gekennzeichnet, daß die optische Einrichtung eine steuerbare Blendeneinrichtung aufweist, welche gleichzeitig mehrere Strahlflecke auf der zu behandelnden Gewebefläche bildet.

5. Vorrichtung nach einem der Ansprüche 1 bis 3, dadurch gekennzeichnet, daß die optische Einrichtung eine steuerbare Blendeneinrichtung aufweist, die einen von der Kreisform abweichenden Strahlfleck auf der zu behandelnden Gewebefläche bildet.

6. Vorrichtung nach Anspruch 4 oder 5, dadurch gekennzeichnet, daß der bzw. die auf der zu behandelnden Gewebefläche gebildete Strahlfleck bzw. Strahlflecke um eine zentrisch verlaufende Achse drehbar sind.

7. Vorrichtung nach einem der Ansprüche 1 bis 6, dadurch gekennzeichnet, daß der auf das zu behandelnde Gewebe auftreffende Laserstrahl eine geringere Strahlfleckfläche auf der Gewebeoberfläche bildet als die zu behandelnde Gewebefläche.

8. Vorrichtung nach einem der Ansprüche 1 bis 7, dadurch gekennzeichnet, daß eine Ablenkeinrichtung vorgesehen ist, mit welcher der Laserstrahl über die zu behandelnde Gewebefläche geführt ist.

9. Vorrichtung nach einem der Ansprüche 1 bis 8, dadurch gekennzeichnet, daß der Laserstrahl impulsförmig ausgebildet ist und seine Auslenkung im Takt einzelner oder mehrerer (zwei oder mehr) Laserstrahlimpulse erfolgt.

10. Vorrichtung nach einem der Ansprüche 1 bis 9, dadurch gekennzeichnet, daß der Laserstrahl bezüglich einer Fixierlinie, entlang welcher das zu behandelnde Auge ausrichtbar ist, ausgelenkt ist.

11. Vorrichtung nach einem der Ansprüche 1 bis 10, dadurch gekennzeichnet, daß der Öffnungsrand der Blende die Form eines Polygons (Vieleckes) hat.

12. Vorrichtung nach einem der Ansprüche 1 bis 11, dadurch gekennzeichnet, daß der Öffnungsbereich der Blendeneinrichtung eine etwa sternförmige Blendenöffnung aufweist.

13. Vorrichtung nach einem der Ansprüche 1 bis 12, dadurch gekennzeichnet, daß der Öffnungsbereich der Blendeneinrichtung eine regelmäßige geometrische Gestalt hat.

14. Vorrichtung nach einem der Ansprüche 1 bis 12, dadurch gekennzeichnet, daß der Öffnungsbereich der Blendeneinrichtung eine unregelmäßige geometrische Gestalt hat.

15. Vorrichtung nach einem der Ansprüche 1 bis 14, dadurch gekennzeichnet, daß die Querschnittsgeometrie der Blendenöffnung durch Fraktals gebildet ist.

16. Vorrichtung nach einem der Ansprüche 1 bis 15, dadurch gekennzeichnet, daß die Fläche des durch die Blendeneinrichtung gebildeten Strahlflecks auf dem zu behandelnden Gewebe etwa 1/30 - 1/10 der zu behandelnden Gewebefläche beträgt.
